(19) **Europäisches Patentamt / European Patent Office / Office européen des brevets**

(11) **EP 4 202 402 A1**

(12) **EUROPEAN PATENT APPLICATION**
published in accordance with Art. 153(4) EPC

(43) Date of publication:
28.06.2023 Bulletin 2023/26

(21) Application number: 22861654.6

(22) Date of filing: 22.08.2022

(51) International Patent Classification (IPC):
$G01N\ 3/00$ (2006.01)    $G01N\ 3/06$ (2006.01)
$G01N\ 3/08$ (2006.01)    $G01N\ 3/24$ (2006.01)
$G01N\ 3/60$ (2006.01)    $G01N\ 33/44$ (2006.01)

(52) Cooperative Patent Classification (CPC):
G01N 3/00; G01N 3/06; G01N 3/08; G01N 3/24;
G01N 3/60; G01N 33/44

(86) International application number:
PCT/KR2022/012494

(87) International publication number:
WO 2023/027444 (02.03.2023 Gazette 2023/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 27.08.2021 KR 20210113913
19.10.2021 KR 20210138918
13.07.2022 KR 20220086176

(71) Applicant: Lg Chem, Ltd.
Seoul 07336 (KR)

(72) Inventors:
• MOON, Sung Nam
Daejeon 34122 (KR)

• KIM, Hyun Tae
Daejeon 34122 (KR)
• JEON, Sang Jin
Daejeon 34122 (KR)
• JUNG, Jin Mi
Daejeon 34122 (KR)
• CHOI, Jin Uk
Daejeon 34122 (KR)
• IM, Dam Hyeok
Daejeon 34122 (KR)

(74) Representative: Goddar, Heinz J.
Boehmert & Boehmert
Anwaltspartnerschaft mbB
Pettenkoferstrasse 22
80336 München (DE)

(54) **SYSTEM AND METHOD FOR PREDICTING PHYSICAL PROPERTIES OF MULTILAYER MATERIAL**

(57) The present technology relates to a system and method for predicting the physical properties of a multilayer material, and the system and method may predict physical properties such as the elastic modulus, shear modulus, and Poisson's ratio of an entire laminate when a multilayer material having a laminated structure is developed, and may predict not only the homogenized robustness of the entire laminate of the multilayer material, but also the stress and strain of each layer at the time of occurrence of external forces including expansion stress considering environmental factors such as a temperature change or a humidity change are generated.

EP 4 202 402 A1

【FIG. 4】

$E^k_{1,2}, \upsilon^k_{1,2}, G^k_{1,2}, \theta^k_{1,2}, Z^k$ INPUT —S11

S13— $[S]^k_{1,2}$ ⟷ $[Q]^k_{1,2}$ —S12

$\theta_k$

$[Q]^k_{x,y}$ —S14

$Z_k$

$[A]_{x,y}, [B]_{x,y}, [D]_{x,y}$ —S15

$[a]_{x,y} \quad [b]_{x,y}$
$[c]_{x,y} \quad [d]_{x,y}$ —S16

h

$/E_{x,y}, /\mathcal{V}_{x,y}, /G_{x,y}$ —S17

**Description**

[Technical Field]

This application claims priority to and the benefit of Korean Patent Application No. 10-2021-0113913, filed on August 27, 2021, Korean Patent Application No. 10-2021-0138918, filed on October 19, 2021, and Korean Patent Application No. 10-2022-0086176, filed on July 13, 2022, the disclosures of which are incorporated herein by reference in their entireties.

The present invention relates to a system and method for predicting the physical properties of a multilayer material, and more particularly, to a system and method for predicting the physical properties of a multilayer material when developing the multilayer material.

[Background Art]

A polymer film refers to a non-fibrous flat plastic molded article, which is light, has a good barrier property, high transparency and is relatively inexpensive, so it is used in many fields such as packaging materials, household goods, electronic devices, automobiles, and aircraft.

For example, synthetic polymers such as polyethylene (PE), polypropylene (PP), polyvinyl chloride (PVC), and polyethylene terephthalate (PET) are processed into a polymer film and widely used in Korea and abroad, and currently, many synthetic polymers are used as materials for polymer films either alone or by blending.

A multilayer film is a composite film in which different types of films are laminated for the purpose of multi-functionality of the film, and various types of multilayer films formed of, for example, the combination of a film having the excellent mechanical property of polyethylene (PE) and the printing aesthetics of cellophane, and nylon and a vinyl alcohol-ethylene copolymer are used as packaging materials.

In the case of developing such a multilayer film as a material, it is necessary to predict physical properties such as the elastic modulus, shear modulus, and the Poisson's ratio of the entire laminate.

When the elastic modulus is referred to as "E," the shear modulus is referred to as "G," and the Poisson's ratio is referred to as "υ," the relationship between them is as follows:

$$G = \frac{E}{2(1+v)}$$

The material has a property in which lateral transformation (transformation in the direction proportional to a load) and longitudinal transformation (transformation in the direction of load) are proportional to each other within the elastic range, and the ratio of these two transformations have a certain value according to a material within the elastic limit and is called the Poisson's ratio.

In addition, when the elastic modulus represents the degree of strains occurring when an elastic material is subjected to stress, and the elastic modulus is referred to as "E," the stress is referred to as "σ," and the strain is referred to as "ε," the relationship between them is as follows:

$$E = \frac{\sigma}{\varepsilon}$$

Meanwhile, since materials for a multilayer film are anisotropic in a machine direction (MD) and a transverse direction (TD) during the manufacturing process, for the robust design for materials, it is necessary to predict not only the homogenized rigidity of the entire laminate of the multilayer film, but also the stress and strain of each film layer when an external force is generated.

Conventionally, after manufacturing a multilayer film by laminating separate films, its physical properties were evaluated. However, for the evaluation of physical properties, the manufacture and evaluation of various combinations of multilayer films are costly in terms of time and money. Therefore, even when the multilayer film is not directly manufactured, there is a high need for a method that can predict its properties in advance.

[Summary]

[Technical Problem]

To solve technical problems of the present invention, the present invention is directed to providing a system and method for predicting the physical properties of a multilayer material, which can predict physical properties such as the elastic modulus, shear modulus, and Poisson's ratio of the entire laminate when a multilayer material having a laminate structure is developed, and predict not only the homogenized robustness of the entire laminate of a multilayer material, but also the stress and strain of each layer at the time of occurrence of an external force including expansion stress considering environmental factors such as a temperature change or a humidity change.

[Technical Solution]

To accomplish the aforementioned purposes, the present invention provides a system for predicting the physical properties of a multilayer material. In one embodiment, the system for predicting physical properties of a multilayer material according to the present invention includes

receive any one or more of an elastic modulus, Poisson's ratio, shear modulus, thickness and lamination angle of each layer, and a total thickness of the multilayer material; and
a calculate the physical properties of the multilayer material based on one or more of the elastic modulus, Poisson's ratio, shear modulus, thickness and lamination angle of each individual layer of the multilayer material, and total thickness of the multilayer material;
wherein the calculated physical properties of the multilayer material include one or more of elastic moduli of the multilayer material in first and second directions, shear moduli of the multilayer material in the first and second directions, or Poisson's ratios ($/\upsilon_{x,y}$) of the multilayer material in the first and second directions.

In some examples, the controller may be configured to receive and calculate any one or more of the elastic moduli ($/E_{x,y}$), shear moduli ($/G_{x,y}$), and Poisson's ratios ($/\upsilon_{x,y}$) of the multilayer material.
In some examples, the present invention provides a method of predicting the physical properties of a multilayer material described herein. In one embodiment, the method includes

receiving any one or more of an elastic modulus, Poisson's ratio, shear modulus, thickness, and lamination angle of each layer, and the total thickness of the multilayer material; and
calculating the physical properties of the multilayer material based on one or more of the elastic modulus, Poisson's ratio, shear modulus, thickness and lamination angle of each individual layer of the multilayer material, and total thickness of the multilayer material, wherein the calculated physical properties of the multilayer material include one or more of elastic moduli of the multilayer material in first and second directions, shear moduli of the multilayer material in the first and second directions, or Poisson's ratios ($/\upsilon_{x,y}$) of the multilayer material in the first and second directions.

[Advantageous Effects]

The present invention can predict physical properties such as the elastic modulus, shear modulus and Poisson's ratio of an entire laminate when developing a multilayer material, and can predict not only the homogenized robustness of the entire laminate of the multilayer material, but also the stress and strain of each layer at the time of occurrence of an external force including expansion stress considering environmental factors such as a temperature change or a humidity change is generated.

[Brief Description of the Drawings]

FIG. 1 is a diagram illustrating a configuration of a system for predicting the physical properties of a multilayer material according to a first embodiment of the present invention.
FIG. 2 is a diagram illustrating a configuration of an input screen of the system for predicting the physical properties of a multilayer material according to the first embodiment of the present invention.
FIG. 3 is a diagram illustrating a configuration of an output screen of the system for predicting the physical properties of a multilayer material according to the first embodiment of the present invention.

FIG. 4 is a flowchart of a method of predicting the physical properties of a multilayer material according to a second embodiment of the present invention.

FIG. 5 is a flowchart of a method of predicting the physical properties of a multilayer material according to a third embodiment of the present invention.

[Detailed Description]

To achieve the above-described purposes, the present invention provides a system for predicting the physical properties of a multilayer material. In one embodiment, the system for predicting the physical properties of a multilayer material having n laminated films (n is an integer of 2 or more) according to the present invention, which includes

an input unit to which values including any one or more of the elastic modulus ($E^k$), Poisson's ratio ($\upsilon^k$), shear modulus ($G^k$), thickness ($Z^k$), and lamination angle ($\theta^k$) of each layer ($k$), and the total thickness ($h$) of the multilayer material are input;

a control unit that calculates the physical properties of the multilayer material by applying the values input to the input unit;

a display that is connected to the control unit; and

a storage unit that is connected to the control unit.

In addition, the control unit processes the values input to the input unit to calculate any one or more of the elastic moduli ($/E_{x,y}$), shear moduli ($/G_{x,y}$), and Poisson's ratios ($/\upsilon_{x,y}$) of the multilayer material.

In an exemplary embodiment, the control unit calculates a stiffness matrix ($[Q]^k$) of each layer ($k$) by applying any one or more of the input elastic modulus ($E^k$), Poisson's ratio ($\upsilon^k$), and shear modulus ($G^k$) of each layer ($k$),

sets an inverse matrix ($[S]^k$) for the stiffness matrix ($[Q]^k$) of each layer ($k$),

resets a stiffness matrix of each layer ($k$) by reflecting the lamination angle ($\theta^k$) of each layer ($k$) in the stiffness matrix ($[Q]^k$),

calculates stiffness matrices ($[A]_{x,y}$, $[B]_{x,y}$, $[D]_{x,y}$) of the multilayer material using the value of the reset stiffness matrix by receiving the thickness information of each layer ($k$),

sets compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material, and

calculates any one or more of the elastic moduli ($/E_{x,y}$), shear moduli ($/G_{x,y}$), and Poisson's ratios ($/\upsilon_{x,y}$) of the multilayer material using the total thickness ($h$) of the multilayer material and the values of the compliance matrices ($[a]_{x,y}$, $[b]_{x,y}$, $[c]_{x,y}$, $[d]_{x,y}$).

In one embodiment, the values input to the input unit includes any one or more of elastic moduli ($E^k_{1,2}$) in the machine direction (MD, 1) and the transverse direction (TD, 2) of each layer ($k$),

Poisson's ratios ($\upsilon^k_{1,2}$) in the machine direction (1) and the transverse direction (2) of each layer ($k$),

shear moduli ($G^K_{1,2}$) in the machine direction (1) and the transverse direction (2) of each layer ($k$),

an angle ($\theta^k$) in the machine direction (1) of each layer with respect to the x direction of the multilayer material, wherein the x direction means one direction arbitrarily set in a plane of the multilayer material, and

a thickness ($Z^k$) of each layer ($k$).

In addition, the control unit processes the values input to the input unit to calculate any one or more of the elastic moduli ($/E_{x,y}$), shear moduli ($/G_{x,y}$), and Poisson's ratios ($/\upsilon_{x,y}$) of the multilayer material.

In another embodiment, the control unit calculates stiffness matrices ($[Q]^k_{1,2}$) in the machine direction (1) and the transverse direction (2) of each layer ($k$) using the elastic moduli ($E^k_{1,2}$), Poisson's ratios ($\upsilon^k_{1,2}$), and shear moduli ($G^k_{1,2}$),

sets inverse matrices ($[S]^k_{1,2}$) for the stiffness matrices ($[Q]^k_{1,2}$) in the machine direction (1) and the transverse direction (2) of each layer ($k$),

resets stiffness matrices ($[Q]^k_{x,y}$) of each layer ($k$) by reflecting a lamination angle ($\theta^k$) of each layer ($k$) in the stiffness matrices ($[Q]^k_{1,2}$),

calculates stiffness matrices ($[A]_{x,y}$, $[B]_{x,y}$, $[D]_{x,y}$) of the multilayer material using the values of the reset stiffness matrices by receiving the thickness information of each layer ($k$),

sets compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material, and

calculates the elastic moduli ($/E_{x,y}$), shear moduli ($/G_{x,y}$), and Poisson's ratios ($/\upsilon_{x,y}$) of the multilayer material using the total thickness (h) of the multilayer material and the values of compliance matrices ($[a]_{x,y}$, $[b]_{x,y}$, $[c]_{x,y}$, $[d]_{x,y}$).

In still another embodiment, the values input to the input unit further include coefficients of thermal expansion ($\alpha^k_{1,2}$) in the machine direction (1) and the transverse direction (2) of each layer (k), coefficients of water expansion ($\beta^k_{1,2}$) in the machine direction (1) and the transverse direction (2) of each layer (k), a temperature change ($\triangle T$), and a humidity change ($\triangle C$). In addition, the control unit processes the values input to the input unit to calculate strains ($\varepsilon^k_{x,y}$) of each layer (k) and stresses ($\sigma^k_{x,y}$) of each layer (k).

In one exemplary embodiment, the system for predicting the physical properties of a multilayer material according to the present invention includes an input unit to which elastic moduli ($E^k_{1,2}$) in the machine direction (1) and the transverse direction (2) of each layer (k), Poisson's ratios ($\upsilon^k_{1,2}$) in the machine direction (1) and the transverse direction (2) of each layer (k), shear moduli ($G^K_{1,2}$) in the machine direction (1) and the transverse direction (2) of each layer (k), an angle ($\theta^k$) in the machine direction (1) of each layer (k) with respect to the x direction of the multilayer material, a thickness ($Z^k$) of each layer (k), coefficients of thermal expansion ($\alpha^k_{1,2}$) in the machine direction (1) and the transverse direction (2) of each layer (k), coefficients of water expansion ($\beta^k_{1,2}$) in the machine direction (1) and the transverse direction (2) of each layer (k), a temperature change ($\triangle T$), and a humidity change ($\triangle C$) are input;

a control unit that is connected to the input unit, and includes a multilayer material property calculation unit and a layer strain and stress calculation unit;
a display that is connected to the control unit; and
a storage unit that is connected to the control unit.

In addition, the control unit calculates stiffness matrices ($[Q]^k_{1,2}$) in the machine direction (1) and the transverse direction (2) of each layer (k) using the elastic moduli ($E^k_{1,2}$), Poisson's ratios ($\upsilon^k_{1,2}$) and shear moduli ($G^k_{1,2}$), sets inverse matrices ($[S]^k_{1,2}$) for the stiffness matrices ($[Q]^k_{1,2}$) in the machine direction (1) and the transverse direction (2) of each layer (k), resets stiffness matrices ($[Q]^k_{x,y}$) of each layer (k) by reflecting a lamination angle ($\theta^k$) of each layer (k) to the stiffness matrices ($[Q]^k_{1,2}$), calculates stiffness matrices ($[A]_{x,y}$, $[B]_{x,y}$, $[D]_{x,y}$) of the multilayer material using the values of the reset stiffness matrices by receiving the thickness information of each layer (k), sets compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material, and calculates any one or more of the elastic moduli ($/E_{x,y}$), shear moduli ($/G_{x,y}$) and Poisson's ratios ($/\upsilon_{x,y}$) of the multilayer material using the total thickness (h) of the multilayer material and the values of the compliance matrices ($[a]_{x,y}$, $[b]_{x,y}$, $[c]_{x,y}$, $[d]_{x,y}$).

In a specific example, the control unit calculates free lamina hydrothermal strains ($e^k_{1,2}$) generated by water expansion of each layer (k) in the main direction of each layer (k) using the coefficients of thermal expansion ($\alpha^k_{1,2}$), coefficients of water expansion ($\beta^k_{1,2}$), temperature change ($\triangle T$) and humidity change ($\triangle C$) of each layer (k),

calculates hygrothermal strain transformations ($e^k_{x,y,s}$) of each layer (k) by reflecting a lamination angle ($\theta^k$) of each layer (k) in the free lamina hydrothermal strains ($e^k_{1,2}$),
calculates hygrothermal forces ($N^{HT}_{x,y,s}$) and hygrothermal moments ($M^{HT}_{x,y,s}$), generated in the multilayer material, based on the hygrothermal strain transformations ($e^k_{x,y,s}$) of each layer (k), the stiffness matrices ($[Q]^k_{x,y}$) of each layer (k), and the thickness ($Z^k$) of each layer (k),
forms total forces ($/N$) and total moments ($1M$) by adding external forces (N, M) to the hygrothermal forces ($N^{HT}_{x,y,s}$) and the hygrothermal moments ($M^{HT}_{x,y,s}$),
calculates strains ($\in^0_{x,y}$) and curvatures ($k_{x,y,s}$) of a middle plane using the total forces ($/N$) and the total moments ($1M$), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material,
calculates strains ($\varepsilon^k_{x,y}$) of each layer (k) by utilizing the strains ($\in^0_{x,y}$) and curvatures ($k_{x,y,s}$) of the middle plane, and the thickness ($Z^k$) information of each layer (k), and
calculates stresses ($\sigma^k_{x,y}$) of each layer (k) using the strains ($\varepsilon^k_{x,y}$) of each layer (k) and the stiffness matrices ($[Q]^k_{x,y}$) of each layer (k).

In addition, the present invention provides a method of predicting the physical properties of a multilayer material. In one embodiment, the method of predicting the physical properties of a multilayer material having n laminated films (n is an integer of 2 or more) according to the present invention includes

inputting any one or more of the elastic modulus ($E^k$), Poisson's ratio ($\upsilon^k$), shear modulus ($G^k$), thickness ($Z^k$), and lamination angle ($\theta^k$) of each layer (k), and the total thickness (h) of the multilayer material; and
calculating any one or more output values of the elastic moduli ($/E_{x,y}$), shear moduli ($/G_{x,y}$) and Poisson's ratios ($/\upsilon_{x,y}$) of the multilayer material by applying the input values.

In an exemplary embodiment, the calculating of output values includes calculating a stiffness matrix ($[Q]^k$) of each layer (k) by applying any one or more of the input elastic modulus ($E^k$), Poisson's ratio ($\upsilon^k$) and shear modulus ($G^k$) of each layer (k);

setting an inverse matrix ($[S]^k$) for the stiffness matrix ($[Q]^k$) of each layer (k);
resetting a stiffness matrix of each layer (k) by reflecting a lamination angle ($\theta^k$) of each layer (k) to the stiffness matrix ($[Q]^k$);
calculating stiffness matrices ($[A]_{x,y}$, $[B]_{x,y}$, $[D]_{x,y}$) of the multilayer material using the values of the reset stiffness matrices by receiving the thickness information of each layer (k);
setting compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material; and
calculating any one or more of the elastic moduli ($/E_{x,y}$), shear moduli ($/G_{x,y}$), and Poisson's ratios ($/\upsilon_{x,y}$) of the multilayer material using the total thickness (h) of the multilayer material and the values of the compliance matrices ($[a]_{x,y}$, $[b]_{x,y}$, $[c]_{x,y}$, $[d]_{x,y}$).

In still another embodiment, in the inputting of input values,

the input values include any one or more of elastic moduli ($E^k_{1,2}$) in the machine direction (MD, 1) and transverse direction (TD, 2) of each layer (k),
Poisson's ratios ($\upsilon^k_{1,2}$) in the machine direction (1) and transverse direction (2) of each layer (k),
shear moduli ($G^K_{1,2}$) in the machine direction (1) and transverse direction (2) of each layer (k),
an angle ($\theta^k$) in the machine direction (1) of each layer with respect to the x direction of the multilayer material, and
a thickness ($Z^k$) of each layer (k).

In a specific example, in the inputting of input values, the input values may further include coefficients of thermal expansion ($\alpha^k_{1,2}$) in the machine direction (1) and the transverse direction (2) of each layer (k), coefficients of water expansion ($\beta^k_{1,2}$) in the machine direction (1) and the transverse direction (2) of each layer (k), a temperature change ($\triangle T$), and a humidity change ($\triangle C$).

In this case, in the calculating of output values, strains ($\varepsilon^k_{x,y}$) of each layer (k) and stresses ($\sigma^k_{x,y}$) of each layer (k) are calculated by processing the values input to the input unit.

In one embodiment, in the method of predicting the physical properties of a multilayer material according to the present invention, the inputting of input values includes inputting elastic moduli ($E^k_{1,2}$) in the machine direction (1) and the transverse direction (2) of each layer (k), Poisson's ratios ($\upsilon^k_{1,2}$) in the machine direction (1) and the transverse direction (2) of each layer (k), shear moduli ($G^K_{1,2}$) in the machine direction (1) and the transverse direction (2) of each layer (k), an angle ($\theta^k$) in the machine direction (1) of each layer (k) with respect to the x direction of the multilayer material, and a thickness ($Z^k$) of each layer (k) (S 1 1).

In another embodiment, in the method of predicting the physical properties of a multilayer material according to the present invention, the calculating of output values includes calculating stiffness matrices ($[Q]^k_{1,2}$) in the machine direction (1) and the transverse direction (2) of each layer (k) using the elastic moduli ($E^k_{1,2}$), Poisson's ratios ($\upsilon^k_{1,2}$) and shear moduli ($G^k_{1,2}$) (S12);

setting inverse matrices ($[S]^k_{1,2}$) for the stiffness matrices ($[Q]^k_{1,2}$) in the machine direction (1) and the transverse direction (2) of each layer (k) (S13);
resetting stiffness matrices ($[Q]^k_{x,y}$) of each layer (k) by reflecting a lamination angle ($\theta^k$) of each layer (k) to the stiffness matrices ($[Q]^k_{1,2}$) (S14);
calculating stiffness matrices ($[A]_{x,y}$, $[B]_{x,y}$, $[D]_{x,y}$) of the multilayer material using the stiffness matrix value reset by receiving the thickness information of each layer (k) (S15);
setting compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material (S16); and
calculating elastic moduli ($/E_{x,y}$), shear moduli ($/G_{x,y}$), and Poisson's ratios ($/\upsilon_{x,y}$) of the multilayer material using the total thickness (h) of the multilayer material and the values of the compliance matrices ($[a]_{x,y}$, $[b]_{x,y}$, $[c]_{x,y}$, $[d]_{x,y}$) (S17).

In another embodiment, in the method of predicting the physical properties of a multilayer material according to the present invention, the inputting of input values includes inputting coefficients of thermal expansion ($\alpha^k_{1,2}$), coefficients of water expansion ($\beta^k_{1,2}$), a temperature change ($\triangle T$), and a humidity change ($\triangle C$) of each layer (k) (S21).

In still another embodiment, in the method of predicting the physical properties of a multilayer material according to the present invention, the calculating of output values includes calculating free lamina hydrothermal strains ($e^k_{1,2}$)

due to water expansion of each layer (k) in the main direction of each layer (k) using the coefficients of thermal expansion ($\alpha^k_{1,2}$), coefficients of water expansion ($\beta^k_{1,2}$), temperature change ($\triangle T$), and humidity change ($\triangle C$) of each layer (k) (S22);

calculating hygrothermal strain transformations ($e^k_{x,y,s}$) of each layer (k) by reflecting a lamination angle ($\theta^k$) of each layer (k) inthe free lamina hydrothermal strains (S23);

calculating hygrothermal forces ($N^{HT}_{x,y,s}$) and hygrothermal moments ($M^{HT}_{x,y,s}$) generated in the multilayer material based on the hygrothermal strain transformations ($e^k_{x,y,s}$) of each layer (k), the stiffness matrices ($[Q]^k_{x,y}$) of each layer (k), and the thickness ($Z^k$) of each layer (k) (S24);

forming total forces (/N) and total moments (/M) by adding external forces (N, M) to the hygrothermal forces ($N^{HT}_{x,y,s}$) and the hygrothermal moments ($M^{HT}_{x,y,s}$) (S25);

calculating strains ($\in^0_{x,y}$) and curvatures ($k_{x,y,s}$) of a middle plane using the total forces (/N) and the total moments (1M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material (S26);

calculating strains ($\varepsilon^k_{x,y}$) of each layer (k) by utilizing the information on the strains ($\in^0_{x,y}$) and curvatures ($k_{x,y,s}$) of the middle plane, and the thickness ($Z^k$) information of each layer (k) (S27); and

calculating stresses ($\sigma^k_{x,y}$) of each layer (k) using the strains ($\varepsilon^k_{x,y}$) of each layer (k) and the stiffness matrices ($[Q]^k_{x,y}$) of each layer (k) (S28).

Hereinafter, to describe the present invention in detail so as to be easily implemented by those of ordinary skill in the art to which the present invention belongs, preferred embodiments of the present invention will be described with reference to the accompanying drawings. Other objects, features, and operational advantages, including the object, action and effect of the present invention will be more apparent by the description of the preferred embodiments.

For reference, embodiments disclosed herein are merely presented by selecting the most preferred embodiments to help the understanding of those of ordinary skill in the art among various possible embodiments, and the technical spirit of the present invention is not necessarily limited only by the presented embodiments. Various changes, additions, and modifications including equivalents or substitutes are possible without departing from the technical spirit of the present invention.

In addition, terms or words used in in the specification and claims should not be construed as being limited to general or dictionary meanings, and should not be interpreted with the meaning and concept in accordance with the technical idea of the present invention based on the principle that the inventors have appropriately defined the concepts of the terms in order to explain the present invention in the best way. In one example, singular expressions include plural expressions unless clearly indicated otherwise in the context, expressions regarding the direction are set based on the position expressed on the drawings for convenience of description, and the expression "connect" or "access" includes not only direct connection or access, but also connection or access through a different component between two components. In addition, the expression "unit" includes a unit implemented using hardware, a unit implemented using software, a unit implemented using both hardware and software, and one unit may be implemented using one or more pieces of hardware or software, and two or more units may be implemented using one piece of hardware or software.

**(First embodiment)**

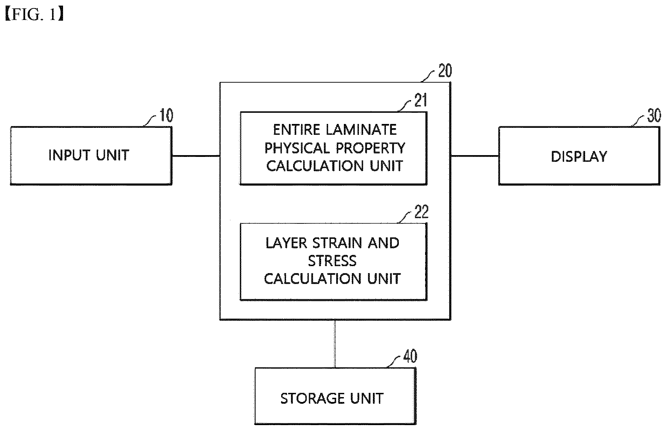

FIG. 1 is a diagram illustrating a configuration of a system for predicting the physical properties of a multilayer material according to a first embodiment of the present invention.

As shown in FIG. 1, the configuration of the system for predicting the physical properties of a multilayer material according to the first embodiment of the present invention is composed of an input unit 10 for a user to input elastic moduli ($E^k_{1,2}$) in a machine direction (MD, hereinafter, set as '1,' denoting a main direction) and a transverse direction (TD, hereinafter, set as '2') of each layer (k), Poisson's ratios ($\upsilon^k_{1,2}$) in the machine direction (1) and transverse direction (2) of each layer (k), shear moduli ($G^K_{1,2}$) in the machine direction (1) and transverse direction (2) of each layer (k), an angle ($\theta^k$) in the machine direction (1) of each layer with respect to the x direction of the multilayer material, a thickness ($Z^k$) of each layer (k), coefficients of thermal expansion ($\alpha^k_{1,2}$) in the machine direction (1) and transverse direction (2) of each layer (k), coefficients of water expansion ($\beta^k_{1,2}$) in the machine direction (1) and the transverse direction (2) of each layer (k), a temperature change ($\triangle T$), and a humidity change ($\triangle C$), a control unit 20, which is connected to the input unit 10 and includes an entire laminate physical property calculation unit 21 and a layer strain and stress calculation unit 22, a display 30 connected to the control unit 20, and a storage unit 40 connected to the control unit 20.

In the present invention, the multilayer material refers to a laminate having a structure in which two or more materials are laminated, such as a multilayer film, for example, a polymer. The multilayer material may refer to a composite material of different materials such as a fiber reinforced plastic (FRP) and an aluminum pouch. For example, the multilayer material may refer to a multilayer film.

Meanwhile, the input unit 10 is for inputting actual physical property values by a user, but the present invention is not necessarily limited thereto. For example, the input unit 10 may access a database (DB) in which information to be input to the input unit 10 is stored.

Moreover, in the Poisson's ratios ($\upsilon^k_{1,2}$) in the machine direction (1) and transverse direction (2) of each layer (k), $\upsilon_{12}$ refers to a Poisson's ratio associated with transformation in the transverse direction (2) when a force is loaded on the material in a machine direction (1), and $\upsilon_{21}$ refers to the Poisson's ratio associated with transformation in the machine direction (1) when the material is loaded in the transverse direction (2). On the other hand, when the material is an anisotropic material, it is possible that $\upsilon_{12}=\upsilon_{21}=\upsilon$.

Meanwhile, the angle ($\theta^k$) may refer to an angle in the counterclockwise direction of each layer with respect to the x-direction of the multilayer material.

In addition, the thickness ($Z^k$) of each layer (k) may refer to the information on coordinates from the thickness center of the entire laminate in the multilayer material.

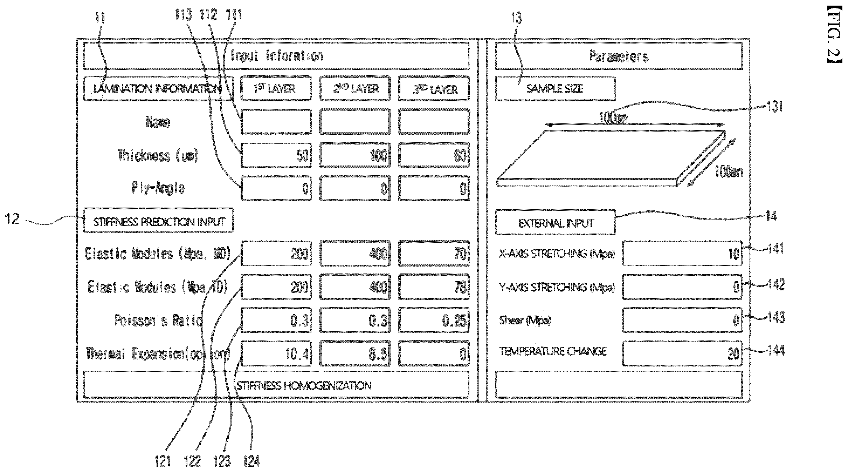

FIG. 2 is a diagram illustrating a configuration of an input screen of the system for predicting the physical properties of a multilayer material according to the first embodiment of the present invention.

As shown in FIG. 2, the configuration of an input screen of the system for predicting the physical properties of a multilayer material according to one embodiment of the present invention includes a lamination information input unit 11 in which a name input field 111, a thickness input field 112, and an angle input field 113 are arranged for each layer; a stiffness prediction input unit 12 in which a machine direction (MD) elastic modulus input field 121, a transverse direction (TD) elastic modulus input field 122, a Poisson's ratio input field 123, and a thermal expansion rate input field 124 are arranged for each layer; a sample size input unit 13 in which a sample width and length input field 131 is arranged; and an external force input unit 14 in which an X-axis tensile force input field 141, a Y-axis tensile force input field 142, a shear force input field 143, and a temperature change input field 144 are arranged.

In the case of the input screen of the first embodiment of the present invention, although the multilayer material is set to have three layers, an input screen having 4 or more layers may be provided.

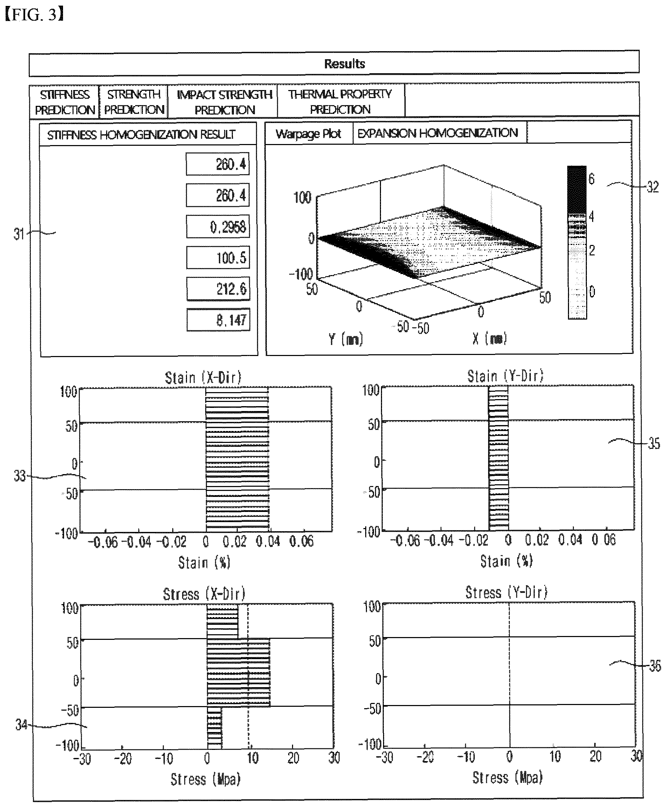

FIG. 3 is a diagram illustrating a configuration of an output screen of the system for predicting the physical properties of a multilayer material according to the first embodiment of the present invention.

As shown in FIG. 3, the output screen of the system for predicting the physical properties of a multilayer material according to the first embodiment of the present invention, as stiffness homogenization results 31, outputs an x-direction elastic modulus, a y-direction elastic modulus, a Poisson's ratio, a shear modulus, a bulk modulus, and a thermal expansion rate, outputs a warpage plot 32, and outputs a thickness-dependent x-direction strain 33, a thickness-dependent x-direction stress 34, a thickness-dependent y-direction strain 35, and a thickness-dependent y-direction stress 36.

**(Second embodiment)**

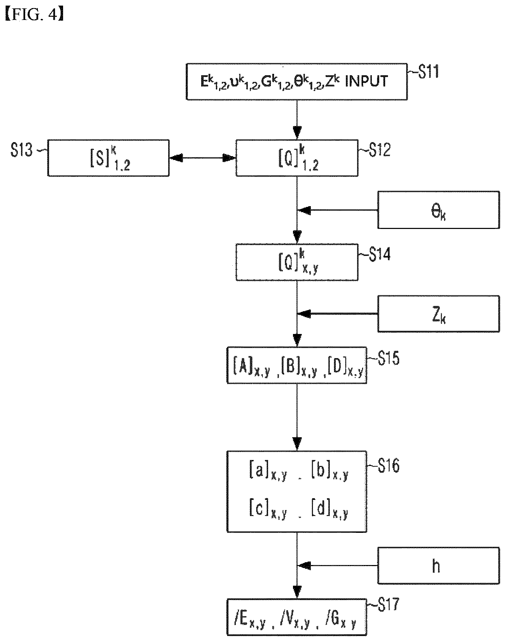

FIG. 4 is a flowchart of a method of predicting the physical properties of a multilayer material according to a second embodiment of the present invention. As shown in FIG. 4, the method of predicting the physical properties of a multilayer material according to the second embodiment of the present invention can be performed as follows.

For the multilayer material having two or more materials laminated, elastic moduli ($E^k_{1,2}$) in the machine direction (1) and transverse direction (2) of each layer (k), Poisson's ratios ($\upsilon^k_{1,2}$) in in the machine direction (1) and transverse direction (2) of each layer (k), shear moduli ($G^k_{1,2}$) in the machine direction (1) and transverse direction (2) of each layer (k), an angle ($\theta^k$) in the machine direction (1) of each layer with respect to the x direction of the multilayer material, and a thickness ($Z^k$) of each layer (k) are input (S11).

Subsequently, using the elastic moduli ($E^k_{1,2}$), Poisson's ratios ($\upsilon^k_{1,2}$), and shear moduli ($G^k_{1,2}$), stiffness matrices ($[Q]^k_{1,2}$) in the machine direction (1) and the transverse direction (2) of each layer (k) are calculated as shown in Equation (1) below (S12).

$$\begin{bmatrix} \sigma_1 \\ \sigma_2 \\ \tau_6 \end{bmatrix} = \begin{bmatrix} Q_{11} & Q_{12} & 0 \\ Q_{12} & Q_{22} & 0 \\ 0 & 0 & Q_{66} \end{bmatrix} \begin{bmatrix} \varepsilon_1 \\ \varepsilon_2 \\ \gamma_6 \end{bmatrix}$$

$$Q_{11} = \frac{E_1}{1 - V_{12} V_{21}} \qquad Q_{22} = \frac{E_2}{1 - V_{12} V_{21}}$$

$$Q_{12} = Q_{21} = \frac{V_{21} E_1}{1 - V_{12} V_{21}} = \frac{V_{12} E_2}{1 - V_{12} V_{21}}$$

$$Q_{66} = G_{12}$$

$$(1)$$

(For an isotropic material, G = E / 2(1+υ))

Inverse matrices ($[S]^k_{1,2}$) for the stiffness matrices ($[Q]^k_{1,2}$) in the machine direction (1) and transverse direction (2) of each layer (k) that have been calculated as described above are set (S13).

The stiffness matrices ($[Q]^k_{x,y}$) of each layer (k) are reset by reflecting a lamination angle ($\theta^k$) of each layer (k) in the obtained stiffness matrices ($[Q]^k_{1,2}$), as shown in Equation (2) below (S14).

$$[T] = \begin{bmatrix} m^2 & n^2 & 2mn \\ n^2 & m^2 & -2mn \\ -mn & mn & m^2 - n^2 \end{bmatrix} \qquad m = \cos\theta, \ n = \sin\theta$$

$$\begin{bmatrix} Q_{xx} & Q_{xy} & 2Q_{xs} \\ Q_{xy} & Q_{yy} & 2Q_{ys} \\ Q_{xs} & Q_{ys} & 2Q_{ss} \end{bmatrix} = [T^{-1}] \begin{bmatrix} Q_{11} & Q_{12} & 0 \\ Q_{12} & Q_{22} & 0 \\ 0 & 0 & 2Q_{66} \end{bmatrix} [T]$$

$$\begin{bmatrix} \sigma_x \\ \sigma_y \\ \tau_s \end{bmatrix} = \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix} \begin{bmatrix} \varepsilon_x \\ \varepsilon_y \\ \gamma_s \end{bmatrix} \qquad \begin{array}{l} \text{Stress-strain relation reflecting angle} \\ \text{(dir-x,y)} \end{array}$$

$$(2)$$

In addition, the stiffness matrices ($[A]_{x,y}$, $[B]_{x,y}$, $[D]_{x,y}$) of the multilayer material, which is the entire laminate, are calculated using the values of the reset stiffness matrices by receiving the thickness information of each layer (k), as shown in Equation (3) below (S15).

$$A_{ij} = \sum_{k=1}^{n} Q_{ij}^k (z_k - z_{k-1}) \qquad B_{ij} = \frac{1}{2} \sum_{k=1}^{n} Q_{ij}^k (z_k^{\ 2} - z_{k-1}^{\ 2})$$

$$D_{ij} = \frac{1}{3} \sum_{k=1}^{n} Q_{ij}^k (z_k^{\ 3} - z_{k-1}^{\ 3})$$

$$(3)$$

In Equation (3), k indicates each layer, and the multilayer material is formed of a total of n layers, wherein n is an integer of 2 to 10.

The compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material, which is the entire laminate, calculated as described above are set as shown in Equation (4) below (S16).

$$\begin{bmatrix} A_{xx} & A_{xy} & A_{xs} & B_{xx} & B_{xy} & B_{xs} \\ A_{yx} & A_{yy} & A_{ys} & B_{yx} & B_{yy} & B_{ys} \\ A_{sx} & A_{sy} & A_{ss} & B_{sx} & B_{sy} & B_{ss} \\ B_{xx} & B_{xy} & B_{xs} & D_{xx} & D_{xy} & D_{xs} \\ B_{yx} & B_{yy} & B_{ys} & D_{yx} & D_{yy} & D_{ys} \\ B_{sx} & B_{sy} & B_{ss} & D_{sx} & D_{sy} & D_{ss} \end{bmatrix}^{-1} = \begin{bmatrix} a_{xx} & a_{xy} & a_{xs} & b_{xx} & b_{xy} & b_{xs} \\ a_{yx} & a_{yy} & a_{ys} & b_{yx} & b_{yy} & b_{ys} \\ a_{sx} & a_{sy} & a_{ss} & b_{sx} & b_{sy} & b_{ss} \\ c_{xx} & c_{xy} & c_{xs} & d_{xx} & d_{xy} & d_{xs} \\ c_{yx} & c_{yy} & c_{ys} & d_{yx} & d_{yy} & d_{ys} \\ c_{sx} & c_{sy} & c_{ss} & d_{sx} & d_{sy} & d_{ss} \end{bmatrix}$$

(4)

Using the total thickness (h) of the multilayer material, and the values of the set compliance matrices ($[a]_{x,y}$, $[b]_{x,y}$, $[c]_{x,y}$, $[d]_{x,y}$), elastic moduli ($/E_{x,y}$), shear moduli ($/G_{x,y}$), and Poisson's ratios ($/\upsilon_{x,y}$) of the multilayer material, which is the entire laminate, are calculated as shown in Equation (5) below (S17).

$$/E_x = \frac{1}{h a_{xx}} \quad /E_y = \frac{1}{h a_{yy}} \quad /G_{xy} = \frac{1}{h a_{ss}}$$

$$/V_{xy} = -\frac{a_{yx}}{a_{xx}} \quad /V_{yx} = -\frac{a_{xy}}{a_{yy}}$$

(5)

In the inputting elastic moduli ($E^k_{1,2}$), Poisson's ratios ($\upsilon^k_{1,2}$), and shear moduli ($G^K_{1,2}$) of each layer (k) (S11), the shear moduli ($G^k_{1,2}$) are calculated from the elastic moduli ($E^k_{1,2}$) and Poisson's ratios ($\upsilon^k_{1,2}$) by a control unit 20 using the following relation between the elastic modulus (E), the shear modulus (G), and the Poisson's ratio ($\upsilon$) of an isotropic material.

$$G = \frac{E}{2(1+v)}$$

Meanwhile, if a user knows the $G_{12}$ value of each layer (k), the corresponding value may be input to Q66 without automatic calculation. However, since the measurement of the shear modulus of each layer (k) is generally very difficult, the test material is assumed to be an isotropic material, and it is assumed that the shear modulus is calculated from the elastic modulus (E) and the Poisson's ratio ($\upsilon$).

**(Third embodiment)**

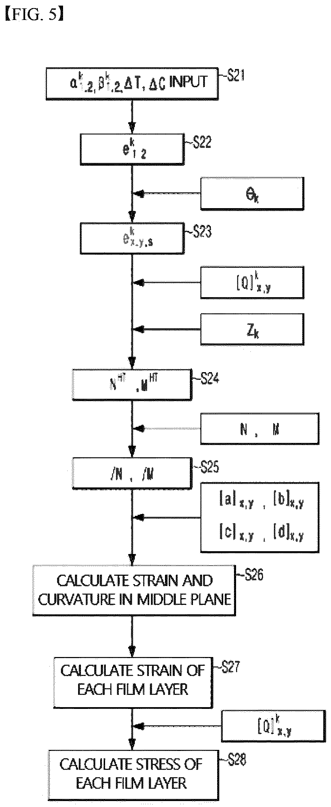

FIG. 5 is a flowchart of a method of predicting the physical properties of a multilayer material according to a third embodiment of the present invention. As shown in FIG. 5, the method of predicting the physical properties of a multilayer material according to a third embodiment of the present invention can be performed as follows.
For the multilayer material having two or more materials laminated,

after inputting coefficients of thermal expansion ($\alpha^k_{1,2}$), coefficients of water expansion ($\beta^k_{1,2}$), a temperature change ($\triangle T$), and a humidity change ($\triangle C$) of each layer (k) (S21),

free lamina hydrothermal strains ($e^k_{1,2}$) caused by water expansion of each layer (k) in the major direction of each layer (k) are calculated using the input coefficients of thermal expansion ($\alpha^k_{1,2}$), coefficients of water expansion ($\beta^k_{1,2}$), temperature change ($\triangle T$), and humidity change ($\triangle C$) of each layer (k), as shown in Equation (6) below (S22).

$$e_1^k = \alpha_1^k \Delta T + \beta_1^k \Delta C \qquad e_2^k = \alpha_1^k \Delta T + \beta_1^k \Delta C \tag{6}$$

Hygrothermal strain transformations ($e_{x,y,s}^k$) of each layer (k) are calculated by reflecting a lamination angle ($\theta^k$) of each layer (k) in the calculated free lamina hydrothermal strains as shown in Equation (7) (S23).

$$e_x^k = e_1^k m^2 + e_2^k n^2 \qquad m = \cos\theta, \; n = \sin\theta$$

$$e_y^k = e_1^k n^2 + e_2^k m^2$$

$$e_s^k = 2(e_1^k + e_2^k) mn \tag{7}$$

Based on the hygrothermal strain transformations ($e_{x,y,s}^k$) of each layer (k), and the stiffness matrices ($[Q]_{x,y}^k$) and the thickness ($Z^k$) of each layer (k), calculated in the second embodiment, hygrothermal forces ($N_{x,y,s}^{HT}$) and hygrothermal moments ($M_{x,y,s}^{HT}$) generated in the multilayer material, which is the entire laminate, are calculated as shown in Equation (8) below (S24).

$$\begin{bmatrix} N_x^{HT} \\ N_y^{HT} \\ N_s^{HT} \end{bmatrix} = \sum_{k=1}^{n} \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix}_k \begin{bmatrix} e_x \\ e_y \\ e_s \end{bmatrix}_k t_k$$

$$\begin{bmatrix} M_x^{HT} \\ M_y^{HT} \\ M_s^{HT} \end{bmatrix} = \sum_{k=1}^{n} \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix}_k \begin{bmatrix} e_x \\ e_y \\ e_s \end{bmatrix}_k z_k t_k \tag{8}$$

Total forces (/N) and total moments (1M) are formed by adding external forces (N, M), which are mechanical loads, to the hygrothermal forces ($N_{x,y,s}^{HT}$) and hygrothermal moments ($M_{x,y,s}^{HT}$) calculated above as shown in Equation (9) below (S25).

$$/N = N + N^{HT} \qquad /M = M + M^{HT} \tag{9}$$

Afterward, strains ($\in_{x,y}^0$) and curvatures ($k_{x,y,s}$) of a middle plane are calculated using the total forces (/N) and the total moments (1M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material, which is the entire laminate, calculated in the second embodiment, as shown in Equation (10) below (S26).

$$
\begin{bmatrix} \epsilon_x^0 \\ \epsilon_y^0 \\ \Upsilon_s^0 \\ \hline K_x \\ K_y \\ K_s \end{bmatrix} = \left[ \begin{array}{ccc|ccc} a_{xx} & a_{xy} & a_{xs} & b_{xx} & b_{xy} & b_{xs} \\ a_{yx} & a_{yy} & a_{ys} & b_{yx} & b_{yy} & b_{ys} \\ a_{sx} & a_{sy} & a_{ss} & b_{sx} & b_{sy} & b_{ss} \\ \hline c_{xx} & c_{xy} & c_{xs} & d_{xx} & d_{xy} & d_{xs} \\ c_{yx} & c_{yy} & c_{ys} & d_{yx} & d_{yy} & d_{ys} \\ c_{sx} & c_{sy} & c_{ss} & d_{sx} & d_{sy} & d_{ss} \end{array} \right] \begin{bmatrix} /N_x \\ /N_y \\ /N_s \\ \hline /M_x \\ /M_y \\ /M_s \end{bmatrix}
$$

$$A \qquad B$$
$$C \qquad D$$

$$(10)$$

By utilizing the strains ($\epsilon^0_{x,y}$) and curvatures ($k_{x,y,s}$) of the middle plane, and the thickness ($Z^k$) information of each layer (k) input through the input unit 10, strains ($\varepsilon^k_{x,y}$) of each layer (k) are calculated as shown in Equation (11) below (S27).

$$
\begin{bmatrix} \varepsilon_x \\ \varepsilon_y \\ \Upsilon_s \end{bmatrix}_k = \begin{bmatrix} \epsilon_x^0 \\ \epsilon_y^0 \\ \Upsilon_s^0 \end{bmatrix} + z_k \begin{bmatrix} k_x \\ k_y \\ k_s \end{bmatrix}
$$

$$(11)$$

In addition, using the strain of each layer (k) and stiffness matrices ($[Q]^k_{x,y}$) of each layer calculated in the second embodiment, stresses ($\sigma^k_{x,y}$) of each layer (k) are calculated as shown in Equation 12 below (S28).

$$
\begin{bmatrix} \sigma_x \\ \sigma_y \\ \Tau_s \end{bmatrix}_k = \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix}_k \begin{bmatrix} \varepsilon_x \\ \varepsilon_y \\ \Upsilon_s \end{bmatrix}_k \qquad (12)
$$

**(Fourth embodiment)**

The actions of the system and method for predicting the physical properties of a multilayer material according to the embodiments of the present invention, which have been configured as above, is as follows.

To predict the physical properties of the multilayer material, a user inputs elastic moduli ($E^k_{1,2}$) in the machine direction (1) and transverse direction (2) of each layer (k, k=1,2,3), Poisson's ratios ($\upsilon^k_{1,2}$) in the machine direction (1) and transverse direction (2) of each layer (k, k=1,2,3), shear moduli ($G^K_{1,2}$) in the machine direction (1) and transverse direction (2) of each layer (k, k=1,2,3), an angle ($\theta^k$) in the machine direction (1) of each layer (k, k=1,2,3) with respect to the x direction of the multilayer material, and a thickness ($Z^k$) of each layer (k) using an input unit 10 while looking at an input screen on a display 30 (S11).

In the input screen provided by the display 30, as shown in FIG. 2, a lamination information input unit 11 in which a name input field 111, a thickness input field 112, and an angle input field 113 are arranged for each layer, a stiffness prediction input unit 12 in which a machine direction (MD) elastic modulus input field 121, a transverse direction (TD) elastic modulus input field 122, a Poisson's ratio input field 123, and a thermal expansion rate input field 124 are arranged for each layer, a sample size input unit 13 in which a sample width and length input field 131 is arranged; and an external force input unit 14 in which an X-axis tensile force input field 141, a Y-axis tensile force input field 142, a shear force input field 143, and a temperature change input field 144 are arranged are provided, information that does not appear on the input screen is automatically calculated by the control unit 20 and stored in a storage unit 40. For example, when the machine direction (MD) elastic modulus ($E^k_1$), transverse direction (TD) elastic modulus ($E^k_2$), and Poisson's ratios ($\upsilon^k_{1,2}$) are input by layer through the input screen and the input unit 10, a control unit 20 automatically calculates shear moduli ($G^k_{1,2}$) using the relationship between an elastic modulus (E), a shear modulus (G) and a Poisson's ratio ($\upsilon$) of an isotropic material below, and then stores the same in the storage unit 40.

$$G = \frac{E}{2(1+v)}$$

Next, an entire laminate physical property calculation unit 21 of the control unit 20 calculates stiffness matrices ($[Q]^k_{1,2}$) in the machine direction (1) and the transverse direction (2) of each layer (k) using the elastic moduli ($E^k_{1,2}$), Poisson's ratios ($\upsilon^k_{1,2}$), and shear moduli ($G^k_{1,2}$) as shown in Equation (1) below (S12).

$$\begin{bmatrix} \sigma_1 \\ \sigma_2 \\ \tau_6 \end{bmatrix} = \begin{bmatrix} Q_{11} & Q_{12} & 0 \\ Q_{12} & Q_{22} & 0 \\ 0 & 0 & Q_{66} \end{bmatrix} \begin{bmatrix} \varepsilon_1 \\ \varepsilon_2 \\ \gamma_6 \end{bmatrix}$$

$$Q_{11} = \frac{E_1}{1 - v_{12} v_{21}} \qquad Q_{22} = \frac{E_2}{1 - v_{12} v_{21}}$$

$$Q_{12} = Q_{21} = \frac{v_{21} E_1}{1 - v_{12} v_{21}} = \frac{v_{12} E_2}{1 - v_{12} v_{21}}$$

$$Q_{66} = G_{12}$$

(1)

In Equation (1), for the isotropic material, G = E / 2(1+$\upsilon$).

Subsequently, the entire laminate physical property calculation unit 21 of the control unit 20 obtains inverse matrices ($[S]^k_{1,2}$) for the calculated stiffness matrices ($[Q]^k_{1,2}$) in the machine direction (1) and the transverse direction (2) of each layer (k) and set as compliance matrices (S13). This is for converting a stiffness matrix, which is a singular matrix, into an invertible matrix in which an inverse matrix is present.

Afterward, the entire laminate physical property calculation unit 21 of the control unit 20 resets stiffness matrices ($[Q]^k_{x,y}$) of each layer (k) by reflecting a lamination angle ($\theta^k$) of the multilayer material in the obtained stiffness matrices ($[Q]^k_{1,2}$) of each layer (k) in the x or y direction, as shown in Equation (2) below (S14).

$$[T] = \begin{bmatrix} m^2 & n^2 & 2mn \\ n^2 & m^2 & -2mn \\ -mn & mn & m^2 - n^2 \end{bmatrix} \quad m=\cos\theta, \; n=\sin\theta$$

$$\begin{bmatrix} Q_{xx} & Q_{xy} & 2Q_{xs} \\ Q_{xy} & Q_{yy} & 2Q_{ys} \\ Q_{xs} & Q_{ys} & 2Q_{ss} \end{bmatrix} = [T^{-1}] \begin{bmatrix} Q_{11} & Q_{12} & 0 \\ Q_{12} & Q_{22} & 0 \\ 0 & 0 & 2Q_{66} \end{bmatrix} [T]$$

$$\begin{bmatrix} \sigma_x \\ \sigma_y \\ \tau_s \end{bmatrix} = \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix} \begin{bmatrix} \varepsilon_x \\ \varepsilon_y \\ \gamma_s \end{bmatrix} \quad \begin{array}{l} \text{Stress-strain relation reflecting angle} \\ (\text{dir-x,y}) \end{array}$$

$$(2)$$

Subsequently, the entire laminate physical property calculation unit 21 of the control unit 20 calculates stiffness matrices ($[A]_{x,y}$, $[B]_{x,y}$, $[D]_{x,y}$) of the multilayer material, which is the entire laminate, using the values of the reset stiffness matrices by receiving the thickness information of each layer (k), as shown in Equation (3) below (S15).

$$A_{ij} = \sum_{k=1}^{n} Q_{ij}^{k}(z_k - z_{k-1}) \quad B_{ij} = \frac{1}{2}\sum_{k=1}^{n} Q_{ij}^{k}(z_k^{\,2} - z_{k-1}^{\,2})$$

$$D_{ij} = \frac{1}{3}\sum_{k=1}^{n} Q_{ij}^{k}(z_k^{\,3} - z_{k-1}^{\,3})$$

$$(3)$$

In Equation (3), k indicates each layer, and the multilayer material is formed of a total of n layers, and n is an integer of 2 to 10.

Next, the entire laminate physical property calculation unit 21 of the control unit 20 sets compliance matrices ($[a]_{x,y}$, $[b]_{x,y}$, $[c]_{x,y}$, $[d]_{x,y}$) for the calculated stiffness matrices ($[A]_{x,y}$, $[B]_{x,y}$, $[D]_{x,y}$) of the multilayer material, which is the entire laminate, as shown in Equation (4) (S16).

$$\begin{bmatrix} A_{xx} & A_{xy} & A_{xs} & B_{xx} & B_{xy} & B_{xs} \\ A_{yx} & A_{yy} & A_{ys} & B_{yx} & B_{yy} & B_{ys} \\ A_{sx} & A_{sy} & A_{ss} & B_{sx} & B_{sy} & B_{ss} \\ B_{xx} & B_{xy} & B_{xs} & D_{xx} & D_{xy} & D_{xs} \\ B_{yx} & B_{yy} & B_{ys} & D_{yx} & D_{yy} & D_{ys} \\ B_{sx} & B_{sy} & B_{ss} & D_{sx} & D_{sy} & D_{ss} \end{bmatrix}^{-1} = \begin{bmatrix} a_{xx} & a_{xy} & a_{xs} & b_{xx} & b_{xy} & b_{xs} \\ a_{yx} & a_{yy} & a_{ys} & b_{yx} & b_{yy} & b_{ys} \\ a_{sx} & a_{sy} & a_{ss} & b_{sx} & b_{sy} & b_{ss} \\ c_{xx} & c_{xy} & c_{xs} & d_{xx} & d_{xy} & d_{xs} \\ c_{yx} & c_{yy} & c_{ys} & d_{yx} & d_{yy} & d_{ys} \\ c_{sx} & c_{sy} & c_{ss} & d_{sx} & d_{sy} & d_{ss} \end{bmatrix}$$

$$(4)$$

Subsequently, the entire laminate physical property calculation unit 21 of the control unit 20 obtains a total thickness (h) of the multilayer material, which is the entire laminate, from the thickness information of each layer (k), and then calculates elastic moduli ($/E_{x,y}$), shear moduli ($/G_{x,y}$), and Poisson's ratios ($/\upsilon_{x,y}$) of the multilayer material, which is the entire laminate, using the values of the compliance matrices ($[a]_{x,y}$, $[b]_{x,y}$, $[c]_{x,y}$, $[d]_{x,y}$) set above, as shown in Equation (5) below (S 17).

$$/E_x = \frac{1}{ha_{xx}} \qquad /E_y = \frac{1}{ha_{yy}} \qquad /G_{xy} = \frac{1}{ha_{ss}}$$

$$/V_{xy} = -\frac{a_{yx}}{a_{xx}} \qquad /V_{yx} = -\frac{a_{xy}}{a_{yy}}$$

$$(5)$$

Next, the user inputs coefficients of thermal expansion ($\alpha^k_{1,2}$), coefficients of water expansion ($\beta^k_{1,2}$), a temperature change ($\triangle T$), and a humidity change ($\triangle C$) of each layer (k) using the input unit 10 while looking at an input screen (not shown) on the display 30 (S21), and then a layer strain and stress calculation unit 22 in the control unit 20 reads the values input.

Subsequently, using the input coefficient of thermal expansion($\alpha^k_{1,2}$), coefficient of water expansion($\beta^k_{1,2}$), temperature change ($\triangle T$), and humidity change ($\triangle C$) of each layer (k), the layer strain and stress calculation unit 22 in the control unit 20 calculates free lamina hydrothermal strains ($e^k_{1,2}$) caused by the water expansion of each layer (k) in the major direction of each layer (k) as shown in Equation (6) below (S22).

$$e^k_1 = \alpha^k_1 \Delta T + \beta^k_1 \Delta C \qquad e^k_2 = \alpha^k_1 \Delta T + \beta^k_1 \Delta C$$

$$(6)$$

Next, the layer strain and stress calculation unit 22 in the control unit 20 calculates hygrothermal strain transformations ($e^k_{x,y,s}$) of each layer (k) by reflecting a lamination angle of each layer (k) in the calculated free lamina hydrothermal strains as shown in Equation (7) below (S23).

$$e^k_x = e^k_1 m^2 + e^k_2 n^2 \qquad\qquad m = \cos\theta, \; n = \sin\theta$$

$$e^k_y = e^k_1 n^2 + e^k_2 m^2$$

$$e^k_s = 2(e^k_1 + e^k_2) mn$$

$$(7)$$

Subsequently, based on the hygrothermal strain transformations ($e^k_{x,y,s}$) of each layer (k), and the stiffness matrices ($[Q]^k_{x,y}$) and thickness ($Z^k$) of each layer (k) obtained in the second embodiment, the layer strain and stress calculation unit 22 in the control unit 20 calculates hygrothermal forces ($N^{HT}_{x,y,s}$) and hygrothermal moments ($M^{HT}_{x,y,s}$), generated in the multilayer material, which is the entire laminate, as shown in Equation (8) below (S24).

This is for obtaining hygrothermal forces ($N^{HT}_{x,y,s}$) and hygrothermal moments ($M^{HT}_{x,y,s}$) generated in the multilayer material, which is the entire laminate, by summing up stresses caused by the hygrothermal strain transformations ($e^k_{x,y,s}$) of each layer (k).

$$\begin{bmatrix} N^{HT}_x \\ N^{HT}_y \\ N^{HT}_s \end{bmatrix} = \sum_{k=1}^{n} \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix}_k \begin{bmatrix} e_x \\ e_y \\ e_s \end{bmatrix}_k t_k$$

$$\begin{bmatrix} M^{HT}_x \\ M^{HT}_y \\ M^{HT}_s \end{bmatrix} = \sum_{k=1}^{n} \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix}_k \begin{bmatrix} e_x \\ e_y \\ e_s \end{bmatrix}_k z_k t_k \tag{8}$$

Next, by adding external forces (N, M), which are mechanical loads, to the calculated hygrothermal forces ($N^{HT}_{x,y,s}$) and hygrothermal moments ($M^{HT}_{x,y,s}$), the layer strain and stress calculation unit 22 in the control unit 20 forms total forces (/N) and total moments (/M) as shown in Equation (9) below (S25).

$$/N = N + N^{HT} \qquad /M = M + M^{HT} \tag{9}$$

Subsequently, the layer strain and stress calculation unit 22 in the control unit 20 calculates strains ($\epsilon^0_{x,y}$) and curvatures ($k_{x,y,s}$) of a middle plane using the total forces (/N) and the total moments (1M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material, which is the entire laminate, calculated in the second embodiment as shown in Equation 10 below (S26).

$$\begin{bmatrix} \epsilon^0_x \\ \epsilon^0_y \\ \gamma^0_s \\ \hline K_x \\ K_y \\ K_s \end{bmatrix} = \left[\begin{array}{ccc|ccc} a_{xx} & a_{xy} & a_{xs} & b_{xx} & b_{xy} & b_{xs} \\ a_{yx} & a_{yy} & a_{ys} & b_{yx} & b_{yy} & b_{ys} \\ a_{sx} & a_{sy} & a_{ss} & b_{sx} & b_{sy} & b_{ss} \\ \hline c_{xx} & c_{xy} & c_{xs} & d_{xx} & d_{xy} & d_{xs} \\ c_{yx} & c_{yy} & c_{ys} & d_{yx} & d_{yy} & d_{ys} \\ c_{sx} & c_{sy} & c_{ss} & d_{sx} & d_{sy} & d_{ss} \end{array}\right] \begin{bmatrix} /N_x \\ /N_y \\ /N_s \\ \hline /M_x \\ /M_y \\ /M_s \end{bmatrix} \tag{10}$$

A     B     C     D

Next, the layer strain and stress calculation unit 22 in the control unit 20 calculates strains ($\varepsilon^k_{x,y}$) of each layer (k) by utilizing strains ($\epsilon^0_{x,y}$) and curvatures ($k_{x,y,s}$) of the middle plane, and the thickness ($Z^k$) information of each layer (k) input by the input unit 10 as shown in Equation 11 below (S27).

$$\begin{bmatrix} \varepsilon_x \\ \varepsilon_y \\ \gamma_s \end{bmatrix}_k = \begin{bmatrix} \epsilon_x^0 \\ \epsilon_y^0 \\ \gamma_s^0 \end{bmatrix} + z_k \begin{bmatrix} k_x \\ k_y \\ k_s \end{bmatrix} \tag{11}$$

Subsequently, the layer strain and stress calculation unit 22 in the control unit 20 calculates stresses ($\sigma^k_{x,y}$) of each layer (k) by using the strains of each layer (k), and the stiffness matrices ($[Q]^k_{x,y}$) of each layer (k) calculated in the second embodiment as shown in Equation (12) below (S28).

$$\begin{bmatrix} \sigma_x \\ \sigma_y \\ \tau_s \end{bmatrix}_k = \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix}_k \begin{bmatrix} \varepsilon_x \\ \varepsilon_y \\ \gamma_s \end{bmatrix}_k \tag{12}$$

The control unit 20 stores the calculated strains ($\varepsilon^k_{x,y}$) and stresses ($\sigma^k_{x,y}$) of each layer (k) in the storage unit 40, and also outputs them onto the display 30.

On an output screen provided by the display 30, as shown in FIG. 3, as stiffness homogenization results 31, a x-direction elastic modulus, a y-direction elastic modulus, a Poisson's ratio, a shear modulus, a bulk modulus, and a thermal expansion rate are output, a 3D warpage plot 32 is output, a thickness-dependent n x-direction strain 33, a thickness-dependent x-direction stress 34, a thickness-dependent y-direction strain 35, and a thickness-dependent y-direction stress 36 are output.

Meanwhile, the machine direction (MD) elastic modulus and the transverse direction (TD) elastic modulus mean in-plane elastic moduli defined based on the entire laminate by the user, and may be generally defined in consideration of a force applied to the final multilayer material. Meanwhile, the major directions (Dir-1, 2, machine direction or transverse direction) of respective layers (k) constituting the final multilayer material do not necessarily coincide with the reference axis (Dir-x,y) of the final multilayer material defined by the user.

In the present invention, since the lamination order or angle may be adjusted according to the physical property required for the design of the multilayer material, a matrix is reset using the angle of each layer input to enable the configuration of such a multilayer design, and it is possible to convert the stiffness matrix of each layer in the reference axis x or y direction of the entire laminated material. Therefore, the elastic modulus output from the output unit may be the physical property with respect to the x or y direction of the final laminated material.

**(Fifth embodiment)**

In one embodiment of the present invention, when the values input to the input unit are not immediately obtained, they may be deduced through a conversion process using other physical property values.

In one embodiment, in the process of inputting the elastic modulus ($E^k$) of each layer (k) and the Poisson's ratio ($\upsilon^k$) of each layer (k), these values can be calculated using one or more physical property values of a Lame's first parameter ($\lambda^k$), a shear modulus ($G^k$) and a bulk elastic modulus ($K^k$). For example, they can be converted into an elastic modulus ($E^k$) and a Poisson's ratio ($\upsilon^k$) using Formulas 1 to 9 below.

When the available combination is ($\lambda^k$, $G^k$), it is expressed by Formula 1 below.

[Formula 1]

$$E^k = \frac{G^k(3\lambda^k + 2G^k)}{\lambda^k + G^k} \quad , \quad \upsilon^k = \frac{\lambda^k}{2(\lambda^k + G^k)}$$

When the available combination is ($\lambda^k$, $E^k$), it is expressed by Formula 2 below.

[Formula 2]

$$v^k = \frac{A - (E^k + \lambda^k)}{4\lambda^k} \quad , \quad E^k$$

When the available combination is $(\lambda^k, \upsilon^k)$, it is expressed by Formula 3 below.

[Formula 3]

$$E^k = \frac{\lambda^k(1 + \lambda^k)(1 - 2\lambda^k)}{\lambda^k} \quad , \quad v^k$$

When the available combination is $(\lambda^k, K^k)$, it is expressed by Formula 4 below.

[Formula 4]

$$E^k = \frac{9K^k(K^k - \lambda^k)}{3K^k - \lambda^k} \qquad v^k = \frac{\lambda^k}{3K^k - \lambda^k}$$

When the available combination is $(G^k, E^k)$, it is expressed by Formula 5 below.

[Formula 5]

$$v^k = \frac{E - 2G}{2G^k} \quad , \quad E^k$$

When the available combination is $(G^k, \upsilon^k)$, it is expressed by Formula 6 below.

[Formula 6]

$$E^k = 2G^k(1 + v^k) \quad , \quad v^k$$

When the available combination is $(G^k, K^k)$, it is expressed by Formula 7 below.

[Formula 7]

$$E^k = \frac{9K^k G^k}{3K^k + G^k} \qquad v^k = \frac{3K^k - 2G^k}{2(3K^k + G^k)}$$

When the available combination is $(K^k, E^k)$, it is expressed by Formula 8 below.

[Formula 8]

$$v^k = \frac{3K^k - E^k}{6K^k} \quad , \quad E^k$$

When the available combination is $(K^k, \upsilon^k)$, it is expressed by Formula 9 below.

[Formula 9]

$$E^k = 3K^k(1 - 2v^k) \quad , \quad v^k$$

The Formulas 1 to 9 above are examples, and it is possible to combine two or more formulas as needed.

[Description of reference numerals]

10: Input unit
20: Control unit
21: Entire laminate physical property calculation unit
22: Layer strain and stress calculation unit
30: Display
40: Storage unit.

**Claims**

1. A system for predicting physical properties of a multilayer material, the system comprising:
a controller configured to:

receive any one or more of an elastic modulus, Poisson's ratio, shear modulus, thickness and lamination angle of each layer, and a total thickness of the multilayer material; and
a calculate the physical properties of the multilayer material based on one or more of the elastic modulus, Poisson's ratio, shear modulus, thickness and lamination angle of each individual layer of the multilayer material, and total thickness of the multilayer material;
wherein the calculated physical properties of the multilayer material include one or more of elastic moduli of the multilayer material in first and second directions, shear moduli of the multilayer material in the first and second directions, or Poisson's ratios ($/\upsilon_{x,y}$) of the multilayer material in the first and second directions.

2. The system of claim 1, wherein the controller is configured to:

for each individual layer of the multilayer material:

calculate a stiffness matrix of the individual layer based on one or more of the input elastic modulus, Poisson's ratio, and shear modulus of the individual layer,
set an inverse matrix for the stiffness matrix of the individual layer, and
reset the stiffness matrix of the individual layer by reflecting the lamination angle of the individual layer in the stiffness matrix of the individual layer,

calculate stiffness matrices of the multilayer material based on the reset stiffness matrices of each individual layer,
for each stiffness matrix of the multilayer material, set a respective compliance matrix, and
calculate the physical properties of the multilayer material based on the total thickness of the multilayer material and the compliance matrices.

3. The system of claim 1, wherein the controller is configured to receive and calculate any one or more of the elastic moduli, shear moduli, or Poisson's ratios of the multilayer material using at least one of:

elastic moduli of each individual layer of the multilayer material in at least one of a machine direction or a transverse direction of the individual layer,

Poisson's ratios of each individual layer of the multilayer material in at least one of the machine direction or the transverse direction of the individual layer,

shear moduli of each individual layer of the multilayer material in at least one of the machine direction or the transverse direction of the individual layer,

an angle of each individual layer of the multilayer material in the machine direction with respect to the first direction of the multilayer material, wherein the first direction is coplanar with the multilayer material, or

a thickness of each individual layer of the multilayer material.

4. The system of claim 3, wherein the controller is configured to:

for each individual layer of the multilayer material:

calculate a stiffness matrix in the machine direction and the transverse direction of the individual layer based on any one or combination of the elastic moduli, Poisson's ratios, and shear moduli,

set an inverse matrix for the stiffness matrix in the machine direction and the transverse direction of the individual layer, and

reset the stiffness matrix of the individual layer by reflecting a lamination angle of the individual layer to the stiffness matrix of the individual layer,

calculate stiffness matrices of the multilayer material based on the reset stiffness matrices of the individual layers and the thickness of each layer,

set a respective compliance matrix for each stiffness matrix of the multilayer material, and

calculate the physical properties based on the total thickness of the multilayer material and the compliance matrices.

5. The system of claim 1, wherein the controller is configured to receive:

coefficients of thermal expansion in the machine direction and the transverse direction of each individual layer of the multilayer material,

coefficients of water expansion in the machine direction and the transverse direction of each individual layer of the multilayer material,

a temperature change, and

a humidity change; and

calculate a strain of each individual layer (k) and a stress of each individual layer of the multilayer materials based on the received coefficients of thermal expansion, coefficients of water expansion, temperature change and humidity change.

6. The system of claim 1, wherein the controller is further configured to:
receive, for each individual layer of the multilayer material:

elastic moduli in a machine direction and a transverse direction of the individual layer,

Poisson's ratios in the machine direction and the transverse direction of the individual layer,

shear moduli in the machine direction and the transverse direction of the individual layer,

an angle of the individual layer in the machine direction with respect to the first direction of the multilayer material,

a thickness of the individual layer,

coefficients of thermal expansion in the machine direction and the transverse direction of the individual layer,

coefficients of water expansion in the machine direction and the transverse direction of the individual layer,

a temperature change, and

a humidity change; and

calculate each of the physical properties of the multilayer material and each of a stress and a strain on each individual layer of the multilayer material.

7. The system of claim 6, wherein the control unit is configured to:

for each individual layer of the multilayer material:

calculate stiffness matrices in the machine direction and the transverse direction of the individual layer based on the elastic moduli, Poisson's ratios, and shear moduli of the individual layer,
set respective inverse matrices for each of the stiffness matrices in the machine direction and the transverse direction of the individual layer, and
reset the stiffness matrices of the individual layer by reflecting a lamination angle of the individual layer in the stiffness matrices of the individual layer,
calculate stiffness matrices of the multilayer material based on the reset stiffness matrices of each individual layer,
for each stiffness matrix of the multilayer material, set a respective compliance matrix, and
calculate the physical properties of the multilayer material based on the total thickness of the multilayer material and the compliance matrices.

8. The system of claim 7, wherein the controller is configured to:

for each individual layer:

calculate a free lamina hydrothermal strain generated by water expansion of the individual layer in a third direction of the individual layer based on the coefficients of thermal expansion of the individual layer, coefficients of water expansion of the individual layer, temperature change and humidity change,
calculate a hygrothermal strain transformation of the individual layer by reflecting a lamination angle of the individual layer in the free lamina hydrothermal strain of the individual layer, and

calculate hygrothermal forces and hygrothermal moments, generated in the multilayer material in the first and second directions, based on the calculated hygrothermal strain transformations of the individual layers, the stiffness matrices of the individual layers, and the thickness of each individual layer,
calculate a total force and a total moment by adding external forces to the calculated hygrothermal forces and the hygrothermal moments,
calculate strains and curvatures of a middle layer of the multilayer material based on the total force and the total moment, and the compliance matrices for the stiffness matrices of the multilayer material,
for each individual layer other than the middle layer of the multilayer material:

calculate strains of the individual layer based on the strains and curvatures of the middle layer, and the thickness of the individual layer, and
calculate stresses of the individual layer based on the strains and the stiffness matrices of the individual layer.

9. A method of predicting physical properties of a multilayer material, comprising:

receiving any one or more of an elastic modulus, Poisson's ratio, shear modulus, thickness, and lamination angle of each layer, and the total thickness of the multilayer material; and
calculating the physical properties of the multilayer material based on one or more of the elastic modulus, Poisson's ratio, shear modulus, thickness and lamination angle of each individual layer of the multilayer material, and total thickness of the multilayer material, wherein the calculated physical properties of the multilayer material include one or more of elastic moduli of the multilayer material in first and second directions, shear moduli of the multilayer material in the first and second directions, or Poisson's ratios ($/\upsilon_{x,y}$) of the multilayer material in the first and second directions.

10. The method of claim 9, further comprising:

for each individual layer of the multilayer material:

calculating a stiffness matrix of the individual layer based on one or more of the input elastic modulus, Poisson's ratio, and shear modulus of the individual layer,
setting an inverse matrix for the stiffness matrix of the individual layer, and
resetting the stiffness matrix of the individual layer by reflecting the lamination angle of the individual layer in the stiffness matrix of the individual layer,

calculating stiffness matrices of the multilayer material based on the reset stiffness matrices of each individual layer,

for each stiffness matrix of the multilayer material, setting a respective compliance matrix, and

calculating the physical properties of the multilayer material based on the total thickness of the multilayer material and the compliance matrices.

11. The method of claim 10, wherein receiving and calculating any one or more of the elastic moduli, shear moduli, or Poisson's ratios of the multilayer material is based on at least one of:

elastic moduli of each individual layer of the multilayer material in at least one of a machine direction or a transverse direction of the individual layer,

Poisson's ratios of each individual layer of the multilayer material in at least one of the machine direction or the transverse direction of the individual layer,

shear moduli of each individual layer of the multilayer material in at least one of the machine direction or the transverse direction of the individual layer,

an angle of each individual layer of the multilayer material in the machine direction with respect to the first direction of the multilayer material, wherein the first direction is coplanar with the multilayer material, or

a thickness of each individual layer of the multilayer material.

12. The method of claim 11, further comprising:

receiving coefficients of thermal expansion in the machine direction and the transverse direction of each individual layer of the multilayer material, coefficients of water expansion in the machine direction and the transverse direction of each individual layer of the multilayer material, a temperature change, and a humidity change; and

calculating a strain of each individual layer (k) and a stress of each individual layer of the multilayer materials based on the received coefficients of thermal expansion, coefficients of water expansion, temperature change and humidity change.

13. The method of claim 9, further comprising:

receiving, for each individual layer of the multilayer material:

elastic moduli in a machine direction and a transverse direction of the individual layer,

Poisson's ratios in the machine direction and the transverse direction of the individual layer,

shear moduli in the machine direction and the transverse direction of the individual layer,

an angle of the individual layer in the machine direction with respect to the first direction of the multilayer material,

a thickness of the individual layer,

coefficients of thermal expansion in the machine direction and the transverse direction of the individual layer,

coefficients of water expansion in the machine direction and the transverse direction of the individual layer,

a temperature change, and

a humidity change; and

calculating each of the physical properties of the multilayer material and each of a stress and a strain on each individual layer of the multilayer material.

14. The method of claim 9, further comprising:

for each individual layer of the multilayer material:

calculating stiffness matrices in the machine direction and the transverse direction of the individual layer based on the elastic moduli, Poisson's ratios, and shear moduli of the individual layer,

setting respective inverse matrices for each of the stiffness matrices in the machine direction and the transverse direction of the individual layer, and

resetting the stiffness matrices of the individual layer by reflecting a lamination angle of the individual layer in the stiffness matrices of the individual layer,

calculate stiffness matrices of the multilayer material based on the reset stiffness matrices of each individual layer,

for each stiffness matrix of the multilayer material, setting a respective compliance matrix, and

calculating the physical properties of the multilayer material based on the total thickness of the multilayer material and the compliance matrices.

15. The method of claim 9, further comprising:

receiving coefficients of thermal expansion in the machine direction and the transverse direction of each individual layer of the multilayer material, coefficients of water expansion in the machine direction and the transverse direction of each individual layer of the multilayer material, a temperature change, and a humidity change; and calculating a strain of each individual layer (k) and a stress of each individual layer of the multilayer materials based on the received coefficients of thermal expansion, coefficients of water expansion, temperature change and humidity change.

16. The method of claim 9, further comprising:

for each individual layer:

calculating a free lamina hydrothermal strain generated by water expansion of the individual layer in a third direction of the individual layer based on the coefficients of thermal expansion of the individual layer, coefficients of water expansion of the individual layer, temperature change and humidity change, and calculating a hygrothermal strain transformation of the individual layer by reflecting a lamination angle of the individual layer in the free lamina hydrothermal strain of the individual layer,

calculating hygrothermal forces and hygrothermal moments, generated in the multilayer material in the first and second directions, based on the calculated hygrothermal strain transformations of the individual layers, the stiffness matrices of the individual layers, and the thickness of each individual layer, calculating a total force and a total moment by adding external forces to the calculated hygrothermal forces and the hygrothermal moments, calculating strains and curvatures of a middle layer of the multilayer material based on the total force and the total moment, and the compliance matrices for the stiffness matrices of the multilayer material, for each individual layer other than the middle layer of the multilayer material: calculating strains of the individual layer based on the strains and curvatures of the middle layer, and the thickness of the individual layer, and calculating stresses of the individual layer based on the strains and the stiffness matrices of the individual layer.

【FIG. 1】

```
                                                    ┌─── 20
                                              ┌──────────── 21
              ┌─── 10          ┌─────────────│──────────┐
  ┌───────────────────┐        │  │ ENTIRE LAMINATE     │        ┌─── 30
  │                   │        │  │ PHYSICAL PROPERTY   │   ┌──────────────────┐
  │   INPUT UNIT      │────────│  │ CALCULATION UNIT    │───│     DISPLAY      │
  │                   │        │  └─────────────────────┘   │                  │
  └───────────────────┘        │                            └──────────────────┘
                               │              ┌──────────── 22
                               │  ┌─────────────────────┐
                               │  │ LAYER STRAIN AND    │
                               │  │ STRESS              │
                               │  │ CALCULATION UNIT    │
                               │  └─────────────────────┘
                               └──────────────┬──────────┘
                                              │
                                       ┌─── 40
                               ┌──────────────────────┐
                               │    STORAGE UNIT      │
                               └──────────────────────┘
```

【FIG. 2】

【FIG. 3】

【FIG. 4】

$E^k_{1,2}, U^k_{1,2}, G^k_{1,2}, \theta^k_{1,2}, Z^k$ INPUT —S11

S13— $[S]^k_{1,2}$ ⟷ $[Q]^k_{1,2}$ —S12

$\theta_k$

$[Q]^k_{x,y}$ —S14

$Z_k$

$[A]_{x,y}, [B]_{x,y}, [D]_{x,y}$ —S15

$[a]_{x,y}$ . $[b]_{x,y}$
$[c]_{x,y}$ . $[d]_{x,y}$ —S16

h

$/E_{x,y}, /V_{x,y}, /G_{x\,y}$ —S17

【FIG. 5】

## INTERNATIONAL SEARCH REPORT

| International application No. |
|---|
| **PCT/KR2022/012494** |

**A. CLASSIFICATION OF SUBJECT MATTER**

**G01N 3/00**(2006.01)i; **G01N 3/06**(2006.01)i; **G01N 3/08**(2006.01)i; **G01N 3/24**(2006.01)i; **G01N 3/60**(2006.01)i; **G01N 33/44**(2006.01)i

According to International Patent Classification (IPC) or to both national classification and IPC

**B. FIELDS SEARCHED**

Minimum documentation searched (classification system followed by classification symbols)

G01N 3/00(2006.01); B29C 70/02(2006.01); E04C 3/02(2006.01); G01N 3/42(2006.01); G06F 17/10(2006.01); G06F 17/50(2006.01); G06F 30/23(2020.01); H01L 27/11556(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 다층 소재(multilayer material), 물성(physical property), 예측(predict), 탄성 계수(modulus of elasticity), 포아송비(Poisson`s ratio), 전단 계수(shear modulus), 두께(thickness), 강성 매트릭스(stiffness matrix), 역행렬(compliance matrix)

**C. DOCUMENTS CONSIDERED TO BE RELEVANT**

| Category* | Citation of document, with indication, where appropriate, of the relevant passages | Relevant to claim No. |
|---|---|---|
| X | KR 10-1780173 B1 (SABIC GLOBAL TECHNOLOGIES B.V.) 19 September 2017 (2017-09-19) See paragraphs [0025]-[0111], claims 1, 11 and 17 and figures 1-8. | 1,3,9,13 |
| A | | 2,4-8,10-12,14-16 |
| A | KR 10-2005-0112788 A (DAEYANG FOUNDATION et al.) 01 December 2005 (2005-12-01) See paragraphs [0020]-[0143], claims 1-4 and figures 2a-7. | 1-16 |
| A | KR 10-2016-0119393 A (INDUSTRY-ACADEMIC COOPERATION FOUNDATION, YONSEI UNIVERSITY) 13 October 2016 (2016-10-13) See claims 1-7 and figure 1. | 1-16 |
| A | JP 6645623 B2 (JFE STEEL CORPORATION) 14 February 2020 (2020-02-14) See paragraphs [0017]-[0044] and figures 1-4. | 1-16 |

| ✓ | Further documents are listed in the continuation of Box C. | ✓ | See patent family annex. |
|---|---|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **23 November 2022** | **24 November 2022** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2022/012494**

C. DOCUMENTS CONSIDERED TO BE RELEVANT

| Category* | Citation of document, with indication, where appropriate, of the relevant passages | Relevant to claim No. |
|---|---|---|
| A | KR 10-2020-0083050 A (SK HYNIX INC. et al.) 08 July 2020 (2020-07-08)<br>See paragraphs [0055]-[0099] and figure 6. | 1-16 |

Form PCT/ISA/210 (second sheet) (July 2022)

**INTERNATIONAL SEARCH REPORT**
Information on patent family members

International application No.

**PCT/KR2022/012494**

| Patent document cited in search report | | | Publication date (day/month/year) | Patent family member(s) | | | Publication date (day/month/year) |
|---|---|---|---|---|---|---|---|
| KR | 10-1780173 | B1 | 19 September 2017 | CN | 106796617 | A | 31 May 2017 |
| | | | | CN | 106796617 | B | 25 September 2018 |
| | | | | EP | 3180193 | A1 | 21 June 2017 |
| | | | | JP | 2018-156689 | A | 04 October 2018 |
| | | | | JP | 2018-503884 | A | 08 February 2018 |
| | | | | JP | 6356339 | B2 | 11 July 2018 |
| | | | | KR | 10-2017-0054564 | A | 17 May 2017 |
| | | | | US | 2017-0371980 | A1 | 28 December 2017 |
| | | | | WO | 2017-027598 | A1 | 16 February 2017 |
| KR | 10-2005-0112788 | A | 01 December 2005 | KR | 10-0612032 | B1 | 11 August 2006 |
| KR | 10-2016-0119393 | A | 13 October 2016 | KR | 10-1677840 | B1 | 21 November 2016 |
| JP | 6645623 | B2 | 14 February 2020 | CA | 3089090 | A1 | 01 August 2019 |
| | | | | EP | 3745286 | A1 | 02 December 2020 |
| | | | | EP | 3745286 | A4 | 24 March 2021 |
| | | | | JP | WO2020-146554 | A1 | 06 February 2020 |
| | | | | US | 2020-0340952 | A1 | 29 October 2020 |
| | | | | WO | 2019-146554 | A1 | 01 August 2019 |
| KR | 10-2020-0083050 | A | 08 July 2020 | None | | | |

Form PCT/ISA/210 (patent family annex) (July 2022)

**EP 4 202 402 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210113913
- KR 1020210138918
- KR 1020220086176